Europäisches Patentamt

European Patent Office

Office européen des brevets

(19)

(11) Numéro de publication: **0 203 871**
A2

## (12) DEMANDE DE BREVET EUROPEEN

(21) Numéro de dépôt: **86401147.3**

(51) Int. Cl.⁴: **G 01 N 25/18,** G 01 N 33/46

(22) Date de dépôt: **29.05.86**

(30) Priorité: **30.05.85 FR 8508156**

(43) Date de publication de la demande: **03.12.86**
**Bulletin 86/49**

(84) Etats contractants désignés: **AT BE CH DE FR GB IT LI
LU NL SE**

(71) Demandeur: **INSTITUT NATIONAL DE LA RECHERCHE
AGRONOMIQUE (INRA), 149, rue de Grenelle,
F-75341 Paris Cedex 07 (FR)**

(72) Inventeur: **Granier, André François, 79 rue Gambetta
"Les Charmettes" Bâtiment A, F-54130 Saint-Max (FR)**

(74) Mandataire: **Phélip, Bruno et al, c/o Cabinet Harlé &
Phélip 21, rue de la Rochefoucauld, F-75009 Paris (FR)**

(54) **Procédé et dispositif pour la mesure du flux des sève brute dans le tronc d'un végétal tel qu'un arbre.**

(57) L'invention est relative à un procédé pour la mesure du flux de sève brute dans le tronc d'un arbre ou autre végétal, et un dispositif pour la mise en œuvre du procédé.

Selon ce procédé, on fait dans le tronc deux trous (5, 6) de faible diamètre, de préférence l'un au-dessus de l'autre et on introduit dans le plus haut au niveau de l'aubier (3) une sonde chauffante (8) équipée d'un thermocouple, et dans le plus bas une sonde non chauffante. La comparaison des températures permet d'obtenir un index du flux K qui donne la valeur et qui est liée au flux u par une loi du type K = A u exp. B, où A et B sont des constantes.

La présente invention est relative à une méthode et à un dispositif pour la mesure du flux de sève brute dans le tronc d'un arbre ou autre végétal.

La mesure de la transpiration constitue un élément essentiel de la compréhension de la physiologie de l'arbre et de la dynamique des transferts d'eau dans les peuplements forestiers. Dans le cadre des recherches menées par l'inventeur dans ce domaine particulier, celui-ci s'est trouvé confronté au problème de la mesure en continu du flux de sève brute dans le tronc des arbres. 11 existe à ce jour de nombreuses méthodes de mesure du flux transpiratoire. On peut citer, parmi les plus répandues la méthode dite "des impulsions de chaleur" décrite notamment par Swanson R.H., "an instrument for detecting sap movement in woody plants", Sta. Pap. Rocky Mt. For. Range Exp. Sta. N° 68, 1962 et "Velocity distribution patterns in ascending xylem sap during transpiration" in flow its Measurement and control in Science and Industry. Et. Rodger and Dowdell. Instrument Society of America, Vol. 1, 1425-30, 1974. On peut également citer le brevet US 4.059.982 qui décrit un appareil pour la mesure des propriétés thermiques des bio-matériaux, ainsi que le brevet français 1.454.539 qui se rapporte à un dispositif pour la mesure de la conductivité thermique de matière en vrac. Par ailleurs dans Physical Review B. Vol. 8, N° 12, 15-12-1973 pages 5860-5865, DAMON traite plus spécifiquement de la conductivité thermique des silices vitreuses à basse température ("Thermal conductivity of vitrous silica at low temperatures.")

Toutefois, aucune de ces méthodes ne présente les spécifications requises pour permettre, à faible coût, de procéder à un échantillonnage de la transpiration des arbres en peuplements forestiers. La méthode des impulsions de chaleur a en particulier deux inconvénients : son caractère ponctuel dans le tronc et son imprécision en condition de transpiration faible.

Le but de l'invention est de fournir une méthode qui soit fiable et précise même pour les faibles débits de sève, tout en ne mettant en oeuvre qu'un matériel simple et peu coûteux.

L'invention fournit donc un procédé de mesure des variations du flux de sève brute dans le tronc d'un végétal, tel qu'un arbre,

2

qui comprend les étapes de mise en place, dans l'aubier, de deux sondes de mesure de température, dont l'une est chauffante et l'autre ne l'est pas, d'envoi dans la sonde chauffante d'un courant électrique d'intensité constante et d'enregistrement de la différence de température entre les deux sondes, celles-ci étant placées dans le même arbre, à une distance l'une de l'autre telle que la chaleur dégagée par la sonde chauffante ne puisse pas influencer de façon appréciable la sonde non chauffante. De préférence, la sonde non chauffante est placée sensiblement sur la même verticale que la sonde chauffante, et en dessous d'elle.

L'invention fournit encore un dispositif pour la mise en oeuvre du procédé et comprenant un circuit de chauffage stabilisé par la sonde chauffante, un dispositif de mesure à thermocouples dont les soudures chaudes et froides sont placées respectivement dans les sondes chauffante et non chauffante, et des moyens d'enregistrement de la tension aux bornes du dispositif de mesure de température. Dans ce dispositif, la sonde chauffante comprend une âme tubulaire rigide sur laquelle est enroulé un fil chauffant, lui-même entouré d'un tube répartiteur de chaleur en matière bonne conductrice de l'électricité, telle qu'aluminium, l'enroulement de fil et le tube répartiteur ayant une longueur à peu près égale à l'épaisseur de l'aubier dans lequel la sonde doit être insérée, et l'âme tubulaire contenant l'un des fils d'alimentation de l'enroulement.

L'invention va maintenant être exposée plus en détail à l'aide d'exemples pratiques, illustrés à l'aide des dessins, parmi lesquels:

- Fig. 1 est une vue schématique d'ensemble du dispositif en coupe partielle;

- Fig. 2 est une coupe de la sonde chauffante;

- Fig. 3 est un diagramme montrant la corrélation entre le débit et la variation de température;

3

- Fig. 4 donne le résultat d'un enregistrement journalier.

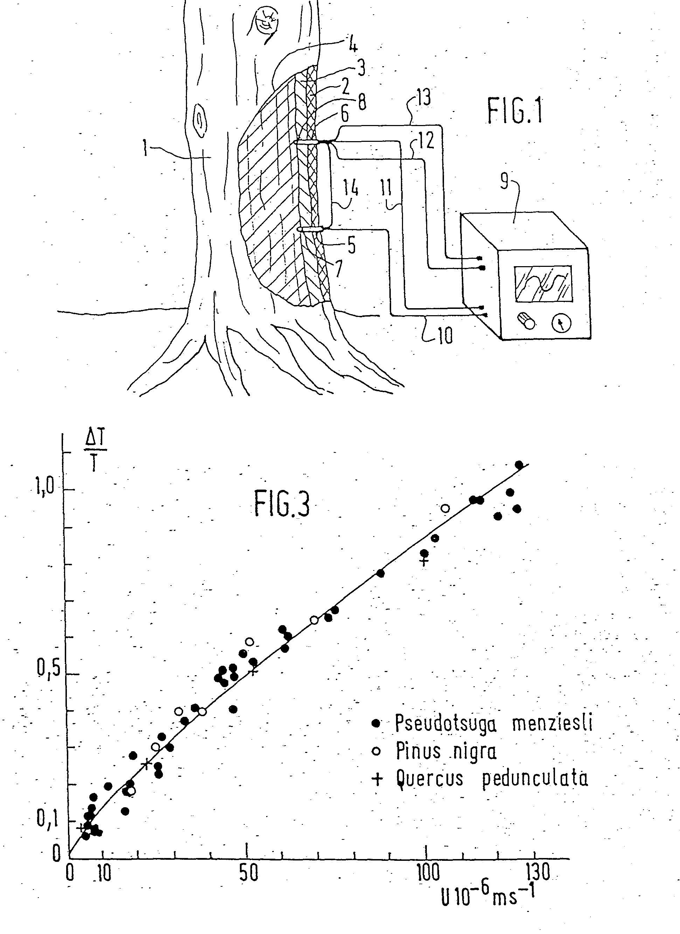

La Fig. 1 montre un arbre 1 qu'on désire étudier. On a représenté en 2 l'écorce, en 3 l'aubier, en 4 le coeur de l'arbre.

Pour la mise en oeuvre du procédé, on pratique dans le tronc, à la perceuse deux trous radiaux 5, 6 de diamètre tel qu'on puisse y introduire les sondes sans jeu, le frottement étant toutefois assez faible pour qu'on puisse éventuellement le récupérer. Les trous doivent traverser l'écorce et l'aubier, mais il est inutile et même déconseillé qu'ils pénètrent dans le coeur. Dans la pratique, le diamètre des trous est de 2 mm environ, et le trou 6, dans lequel on introduira la sonde chauffante est à 50 mm environ au-dessus du trou 5, qui contiendra la sonde non chauffante 7. Le repère 9 désigne le coffret d'alimentation et d'enregistrement, relié aux sondes par des câbles de mesure de température 10, 11 et à la sonde chauffante par des câbles d'alimentation 12, 13. Un câble 14 en constantan relie les deux soudures des thermocouples situés dans les sondes.

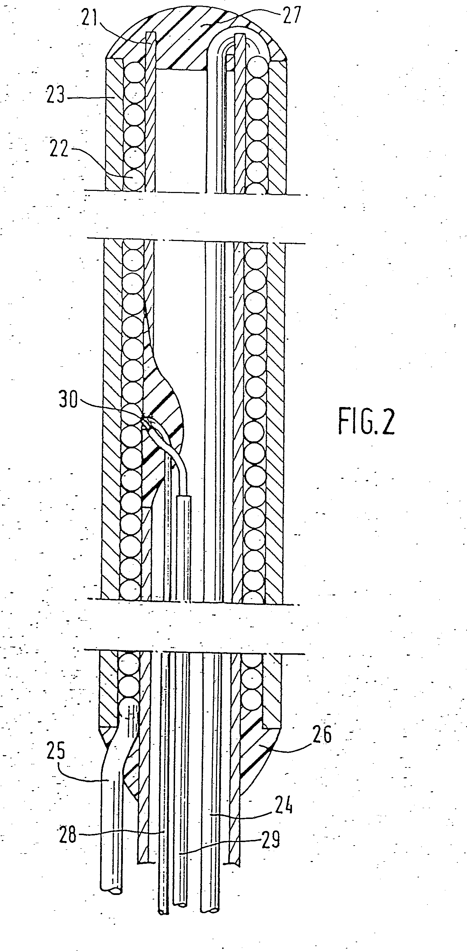

La Fig. 2 est une coupe de la sonde chauffante 8. L'âme 21 de cette sonde est constituée par un tube métallique fin, du type de ceux des aiguilles d'injection à usage médical. A sa partie terminale, l'âme 21 est entourée par un enroulement 22 formé d'environ 75 tours de fil chauffant en constantan isolé avec du verre. Cet enroulement est maintenu par un tube d'aluminium 23, qui est serré étroitement autour de l'enroulement et sert en même temps de répartiteur de température. On notera que dans l'exemple décrit la longueur de ce tube est de 20 mm, pour un diamètre extérieur de 2 mm. Il paraît difficile d'aller plus loin dans la miniaturisation sans prendre de risques en ce qui concerne la robustesse. La longueur de 20 mm correspond à

4

l'épaisseur de l'aubier 3 des arbres·objets de l'étude. Un des fils 24 d'alimentation de l'enroulement passe à l'intérieur de l'âme 21, l'autre 25 est extérieur. Des masses de colle époxy 26, 27 maintiennent l'enroulement et le tube 23 en place sur l'âme, l'une d'elles sert également à obturer l'extrémité de l'âme. L'intérieur de l'âme livre également passage à deux fils 28, 29 de thermocouple, l'un en cuivre émaillé, l'autre en constantan. Le fil 28 est relié par le câble 10 au coffret d'enregistrement, et le fil 29 est relié par le câble 14 à un fil analogue de la sonde non chauffante 7. Cette sonde non chauffante est identique à la sonde chauffante, mais ses fils 24 et 25 ne sont pas branchés. On évite ainsi une différence de comportement thermique.

Le sondeur 30 entre les extrémités des deux fils 28, 29 est placé dans une petite fenêtre pratiquée dans la paroi de l'âme, à peu près au milieu de la longueur de l'enroulement, et elle y est immobilisée par une masse de colle époxy.

En condition de régime thermique établi entre l'élément chauffant et le milieu (bois + sève), et pour un flux de sève constant, nous supposerons que l'apport de chaleur par effet Joule est égal à la quantité de chaleur dissipée au niveau de la paroi du capteur. On a donc:

$$h \, S \, (T - T_b) = R \, i^2 \qquad (1)$$

avec:

$h$ = coefficient de transfert de la chaleur ($W.m^{-2}.c^{-1}$),

$S$ = aire de la surface d'échange (m2),

$T$ = température du cylindre (°C),

$T_b$ = température du matériau bois en l'absence de chauffage (°C),

$R$ = résistance électrique ($\Omega$),

$i$ = intensité du courant électrique (A)

On admet que le coefficient $h$ est relié au débit de la sève $u$ ($m.s^{-1}$) par une équation de la forme:

5

$$h = h_o (1 + \alpha .u) \qquad (2)$$

où $h_o$ est le coefficient d'échange lorsque $u = 0$ (transpiration nulle), qu'il est possible de calculer d'après (1):

$$h_o = \frac{R.i^2}{S (T_M - Tb)} \qquad (3)$$

$T_M$ désigne la température à flux de sève nul ($u = 0$)

Lorsque u est constant et non nul, il vient:

$$u = \frac{1}{\alpha} \frac{T_M - T}{T - Tb} \qquad (4)$$

Le rapport $\frac{T_M - T}{T - Tb}$ est un nombre sans dimension, que nous appellerons index de flux K, qui est lié à u par une équation du type $K = A.u \exp B$, où A et B sont des constants.

Le capteur a été testé et étalonné sur des fragments de troncs de diamètre compris entre 40 et 50 mm. On a fait circuler de l'eau sous pression dans ces échantillons, et mesuré simultanément le débit de l'eau par pesée de l'exsudat et le signal délivré par le capteur thermique. Le débit pouvait être modifié en ajustant la pression de l'eau. Après les mesures, chaque échantillon était sectionné pour mesurer la section du bois d'aubier au niveau de l'élément chauffant. L'étalonnage a porté sur trois essences différentes: Douglas, Pin noir, et Chêne pédonculé.

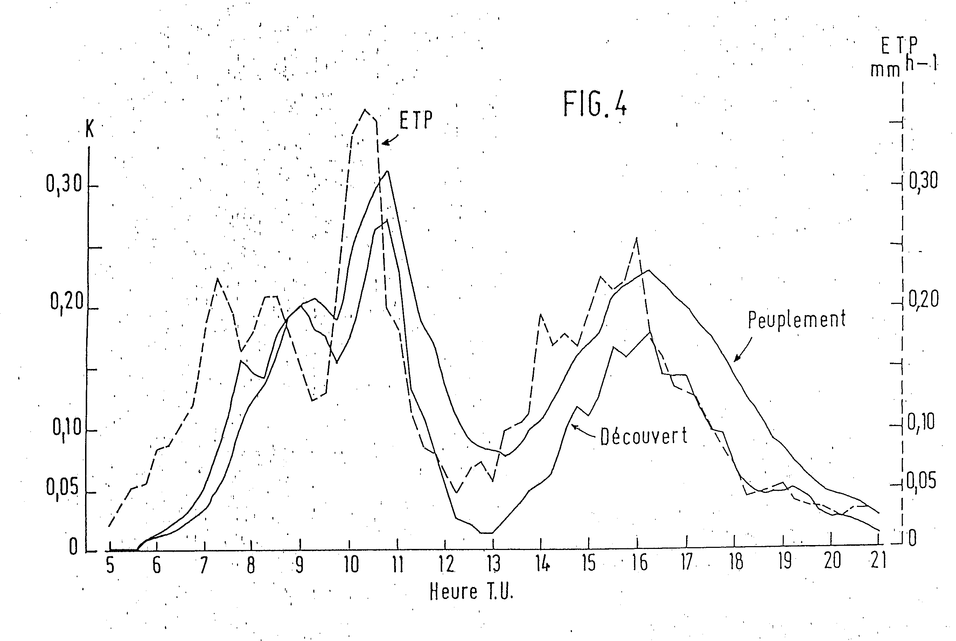

La Fig. 3 représente les résultats obtenus avec en abscisse le flux d'eau par unité de surface u (en $m.s^{-1}$) et en ordonnée le rapport K. Le calcul de K s'effectue d'après la relation (4) en connaissant pour chaque échantillon la température $T_M$ atteinte lorsque le flux d'eau est nul. Il est intéressant de constater que la relation entre K et u est la même pour les trois espèces: le coefficient $\alpha$ de l'équation (4) semble donc, dans les conditions expérimentales, indépendant de l'essence. Un ajustement non linéaire a conduit à la relation expérimentale:

$$K = 0.0206 \ u^{0.8124} \qquad (5) \quad r^2 = 0.96$$
$$n = 53 \text{ points}$$

où u est exprimé en $10^{-6} \text{m.s}^{-1}$.

Signalons enfin que l'intensité du courant dans la résistance chauffante était fixée à une valeur de 0,140 A, ce qui est un compromis entre la sensibilité du capteur (qui augmente avec l'intensité appliquée) et le risque d'un échauffement de la sonde de référence de la température.

Des enregistrements journaliers de flux de transpiration ont été effectués sur des Douglas de la forêt domaniale d'Amance (à 15 km à l'Est de Nancy). On avait choisi des arbres placés dans des situations différentes: des arbres de plein découvert, d'une hauteur de 5 m, et des arbres d'une plantation régulière âgée de 20 ans, pour une hauteur moyenne de 15 m. Quatre arbres (deux dans chacune des situations) ont été équipés chacun d'un capteur thermique inséré radialement dans leur bois d'aubier.

Le coefficient K repose sur l'évaluation de $T_M$ (voir équation (4) au § 1.2). Nous avons supposé que cette température d'équilibre à flux de sève nul pouvait être mesurée lors de séquences nocturnes où l'humidité de l'air est voisine de la saturation.

La Fig. 4 reporte pour une journée la variation du coefficient K, observée pour un arbre de chaque traitement, ainsi que l'évolution de l'évapotranspiration potentielle (E.T.P.) calculée par la formule de Penmann. Nous avons choisi une journée présentant des passages nuageux qui permettent de mettre en évidence la réponse des capteurs. Les deux arbres suivent une évolution

7

similaire, en particulier au niveau des maximas et des minimas. Le retard important de K par rapport à E.T.P. le matin correspond à une phase d'évaporation de la rosée sur les aiguilles. Après cette phase, le facteur K des deux arbres suit la variation de E.T.P. Il est à noter que la chute brutale de E.T.P. intervenant en milieu de journée (passages nuageux) se manifeste de façon plus accentuée pour l'arbre situé en plein découvert. L'arbre de peuplement semble ainsi mieux tamponner les variations de l'E.T.P. En fin de journée, si les deux arbres voient diminuer K en même temps que E.T.P., un retard se manifeste pour le Douglas du peuplement.

Un nombre important de journées a ainsi été étudié en fonction de l'E.T.P. Le coefficient $K_{24}$, égal à la moyenne journalière de K défini d'après l'équation (4) a été calculé. On a constaté qu'il est sensiblement proportionnel à E.T.P. tant que l'arbre étudié est en permanence bien alimenté en eau; la transpiration et donc le coefficient $K_{24}$ sont donc sensiblement proportionnels à E.T.P. Toutefois, certaines des journées présentant des épisodes pluvieux diurnes s'écartent de la règle, ce qui s'interprète ainsi: l'interception de l'eau par le feuillage provoque un blocage de la transpiration; l'énergie incidente est alors utilisée pour évaporer l'eau interceptée.

La méthode que nous avons décrite présente un certain nombre d'avantages, qui permettent d'obtenir des résultats fiables, à deux niveaux:

- au niveau du fonctionnement hydrique de l'arbre, par l'étude fine des variations journalières du flux de sève, particulièrement en période de stress hydrique;

- au niveau du fonctionnement hydrique des peuplements forestiers, sachant que la simplicité de mise en oeuvre et le faible coût de cette technique permet

8

d'aborder la mesure quantitative de la transpiration et de sa variabilité spatiale en forêt.

L'utilisation de cette méthode suppose, lorsqu'on désire calculer le flux total, de connaître la section du bois d'aubier au niveau du point de mesure. Selon le type d'essence et la précision souhaitée, on peut estimer la section du bois d'aubier grâce à un ou plusieurs sondages à la tarière de Pressler ou la mesurer directement après abattage de l'arbre.

0203871

9

REVENDICATIONS

1. Procédé de mesure des variations du flux de sève brute dans le tronc d'un végétal tel qu'un arbre, caractérisé en ce qu'il comprend les étapes de mise en place, dans l'aubier, de deux sondes de mesure de température, dont l'une (8) est chauffante et l'autre (7) ne l'est pas, d'envoi dans la sonde chauffante (8) d'un courant électrique d'intensité constante et d'enregistrement de la différence de température entre les deux sondes, celles-ci étant placées dans le même arbre, à une distance l'une de l'autre telle que la chaleur dégagée par la sonde chauffante (8) ne puisse pas influencer de façon appréciable la sonde non chauffante (7).

2. Procédé selon la revendication 1, caractérisé en ce que la sonde non chauffante (7) est placée sensiblement sur la même verticale que la sonde chauffante, et en dessous d'elle.

3. Dispositif pour la mise en oeuvre du procédé selon l'une des revendications 1 ou 2, et comprenant un circuit de chauffage stabilisé par la sonde chauffante (8), un dispositif de mesure à thermocouples dont les soudures chaudes et froides sont placées respectivement dans les sondes chauffante (8) et non chauffante (7), et des moyens d'enregistrement de la tension aux bornes du dispositif de mesure de température, caractérisé en ce que la sonde chauffante (8) comprend une âme tubulaire rigide (21) sur laquelle est enroulé un fil chauffant (22), lui-même entouré d'un tube répartiteur de chaleur (23) en matière bonne conduction de l'électricité, telle qu'aluminium, l'enroulement de fil (22) et le tube répartiteur (23) ayant une longueur à peu près égale à l'épaisseur de l'aubier dans lequel la sonde doit être insérée, et l'âme tubulaire (21) contenant l'un des fils d'alimentation de l'enroulement (22).

0203871

1/3

FIG.1

FIG.3

- ● Pseudotsuga menziesli
- ○ Pinus nigra
- + Quercus pedunculata

0203871

2/3

FIG.2

FIG.4